# EUROPEAN PATENT SPECIFICATION

(11) **EP 1 399 158 B1**
(45) Date of publication and mention of the grant of the patent: **02.05.2012**
(21) Application number: 02722304.9
(22) Date of filing: 02.05.2002
(51) Int. Cl.: A61K 31/4196, A61K 31/41, C07D 233/61, C07D 233/56, C07D 249/08

(54) **NON-THERAPEUTIC METHOD OF SUPPRESSING OESTRUS IN MAMMALIAN NON-HUMAN ANIMAL**
NICHT-THERAPEUTISCHE METHODE ZUR UNTERDRÜCKUNG VON OESTRUS BEI TIEREN
METHODE NON-THERAPEUTIQUE POUR LA SUPPRESSION DE L' OESTRUS CHEZ DES ANIMAUX

(30) Priority: 02.05.2001 FI 20010905
(43) Date of publication of application: 24.03.2004
(73) Proprietor: Vetcare Oy, 24101 Salo (FI)
(72) Inventor: HEINONEN, Kalevi, FIN-24101 Salo (FI)
(74) Representative: Sundman, Patrik Christoffer
(86) International application number: PCT/FI2002/000373
(87) International publication number: WO 2002/087571

(56) References cited:
- WO-A1-02/09703
- WO-A1-94/13645
- WO-A2-99/30708
- US-A- 5 112 845
- US-A- 5 227 393
- US-A- 5 352 795
- US-A- 5 473 078
- K.J. TURNER ET AL.: 'Effect of chronic administration of an aromatase inhibitor to adult male rats on pituitary and testicular function and fertility' JOURNAL OF ENDOCRINOLOGY vol. 164, 2000, pages 225 - 238, XP002954516
- EMILIE F. RISSMAN ET AL.: 'Effect of vorozole, an aromatase enzyme inhibitor, on sexual behavior, aromatase activity and neural immunoreactivity' JOURNAL OF NEUROENDOCRINOLOGY vol. 8, 1996, pages 199 - 210, XP002954517

## Description

### FIELD OF THE INVENTION

This invention relates to the non therapeutic method of suppressing oestrus in a non-human mammal by administering an aromatase inhibitor.

### BACKGROUND OF THE INVENTION

Estrogen, secreted by ovarian follicles, is responsible of oestrus signs in the bitch and queen. Prevention of estrogen synthesis causes cessation of oestrus behaviour and other signs connected with heat in females. Control of canine and feline oestrus is an important tool in a serious problem of pet overpopulation. In canines, the female oestrus also causes unpredictable behaviour in the males of the surrounding population, this being a problem for especially working dogs, such as service dogs and dog aides.

Alternatives for oestrus prevention at the moment include surgical involvement and gestagen medication. Surgery has limited use in practical, population based control systems. Surgical treatment also has unwanted side effects, one disadvantage being urinary incontinency. The use of gestagen medication is limited because of its side effects in treated animals. Side effects include pyometra and other genital infections.

Aromatase inhibiting compounds for therapeutic applications have been discussed in WO 94/13645 describing the use of selective aromatase inhibiting compounds in treating estrogen dependent diseases. WO 99/30708 proposes using aromatase inhibitor in the treatment of destrusor urethal dyssynergia in men or in decrease an androgen to estrogen ratio in men.

Thus, there is a great need for improved means for the suppression of oestrus in mammals.

### SUMMARY OF THE INVENTION

Surprisingly it has now been found that selective aromatase inhibitors can successfully be used for the suppression of oestrus in non-human mammals.

Thus, this invention relates to a non-therapeutic method of suppressing oestrus in a mammalian non-human animal, comprising administering an effective amount of an aromatase inhibitor of formula (I) 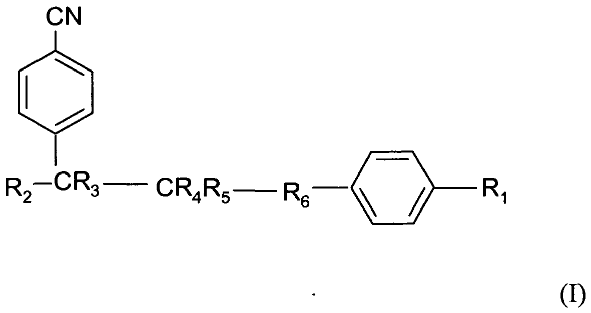 wherein R₁ is hydrogen, methyl, methoxy, nitro, amino, cyano, trifluoromethyl, difluoromethyl, monofluoromethyl or halogen; R₂ is a heterocyclic radical selected from 1-imidazolyl, triazolyl, tetrazolyl, pyrazolyl, pyrimidinyl, oxazolyl, thiazolyl, isoxazolyl and isothiazolyl; R₃ is hydrogen or hydroxy; R₄ is hydrogen; R₅ is hydrogen or hydroxy; or R₄ is hydrogen and R₃ and R₅ combined form a bond; or R₃ is hydrogen and R₄ and R₅ combined form =O; R₆ is methylene, ethylene, -CHOH-, -CH₂CHOH-, - CHOH-CH₂-, -CH=CH- or -C(=O)-; or R₄ is hydrogen and R₅ and R₆ combined is =CH- or =CH-CH₂-; or a stereoisomer, or a non-toxic pharmaceutically acceptable acid addition salt thereof, or a mixture thereof, for the preparation of a veterinary drug useful for suppression of oestrus in a mammal.

### DETAILED DESCRIPTION OF THE INVENTION

Aromatase is an enzyme complex involving a NADPH-cytochrome C reductase and a specific cytochrome P-450 protein. The reaction which is catalyzed by aromatase is unique in the biosynthesis of steroids, as it involves conversion of ring A of the steroid structure to an aromatic ring with the loss of the angular C-19 methyl group and cis-elimination of the 1β and 2β hydrogens to yield estrogen and formic acid. Aromatization is the last and critical step in the biosynthesis of estrogens from cholesterol. Therefore, specific blockade of this enzyme does not cause deprivation of other essential steroids.

All presently described specific aromatase inhibitors have been intended mainly for the treatment of female breast cancer where estrogens stimulate the tumor growth, and aromatase inhibitor, by depleting estrogens, inhibits the tumor growth.

Aromatase inhibitors have also been used in the treatment of detrusor urethral sphincter dyssynergia in men.

Aromatase is needed in the androgen turnover to estrogens. In the connection with this invention, it was found that inhibition of this enzyme causes a decrease in estrogen synthesis in animals and, in consequence, the estrogen dependant oestrus signs.

A suitable group of selective aromatase inhibitors is represented by the compounds covered by formula (I) in International patent application publication No. WO 94/13645. Said compounds 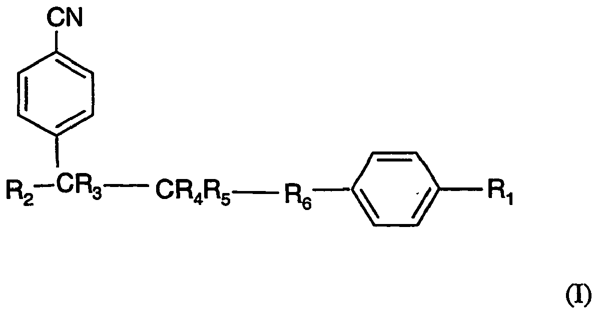 include members wherein R₁ is hydrogen, methyl, methoxy, nitro, amino, cyano, trifluoromethyl, difluoromethyl, monofluoromethyl or halogen; R₂ is a heterocyclic radical selected from 1-imidazolyl, triazolyl, tetrazolyl, pyrazolyl, pyrimidinyl, oxazolyl, thiazolyl, isoxazolyl and isothiazolyl; R₃ is hydrogen or hydroxy; R₄ is hydrogen; R₅ is hydrogen or hydroxy; or R₄ is hydrogen and R₃ and R₅ combined form a bond; or R₃ is hydrogen and R₄ and R₅ combined form =O; R₆ is methylene, ethylene, -CHOH-, -CH₂CHOH-, -CHOH-CH₂-, -CH=CH- or -C(=O)-; or R₄ is hydrogen and R₅ and R₆ combined is =CH- or =CH-CH₂-; or a stereoisomer, or a non-toxic pharmaceutically acceptable acid addition salt thereof.

A preferred compound of this group is 1-[1-(4-cyanophenyl)-3-(4-fluorophenyl)-2-hydroxypropyl]-1,2,4-triazole. Particularly preferred is the compound 1-[1-(4-cyanophenyl)- 3-(4-fluorophenyl)-2-hydroxypropyl]-1,2,4-triazole, diasteroisomer a + d, which also is known under the generic name finrozole. The separated a and d isomers of this diastereomer mixture are also preferred.

The selectivity of the compounds of formula (I) is very high compared to known aromatase inhibitors of the prior art. Very low concentrations of the compounds of formula (I) give rise to aromatase inhibition while very high concentrations would be needed to cause the undesired desmolase effect.

In European Journal of Pharmaceutical Sciences 11 (2000) 109-131, Karjalainen et al. present aromatase and desmolase activities for three compounds of formula I and for certain other aromatase inhibitors. This study reveals that Letrozole and CGS 18320B are far less selective aromatase inhibitors, because already rather low concentrations of the compounds cause the unwanted desmolase activity. Letrozole and CGS18320B are the compounds of prior art, which are structurally closest related to the compounds of formula (I).

For the purpose of this invention, the aromatase inhibitor or its stereoisomer or pharmaceutically acceptable salt can be administered by various routes.

The suitable administration forms include, for example, oral formulations; parenteral injections including intravenous, intramuscular, intradermal and subcutaneous injections; and transdermal or rectal formulations. Suitable oral formulations include e.g. conventional or slow-release tablets and gelatine capsules, and especially liquid mixtures.

The required dosage of the aromatase inhibitor compounds will vary, e.g. with the particular mammal to be treated, the duration of the treatment, and the administration route and the specific compound being employed.

Generally, the treatment should last from the first day of oestrus and should be stopped as soon as the signs have disappeared. For example, finrozole can be administered perorally preferentially once daily. A daily dose is estimated to 0.1-1000 mg/kg body weight, advantageously 0.5-100 mg/kg body weight, preferably 1-50 mg/kg body weight and most preferably 3-15 mg/kg body weight. Finrozole can be given as tablets or other formulations like gelatine capsules alone or mixed in any clinically acceptable non-active ingredients, which are used in the pharmaceutical industry.

The invention can be used for suppressing oestrus symptoms in any species of non-human mammals, especially domestic pets including dogs, cats, mice, rabbits, guinea pigs and the like.

The invention will be illuminated by the following, non-restrictive Experimental Section.

### EXPERIMENT

A medical trial was conducted at University of Kuopio with six (6) beagle bitches the average weight of which was 10 kg and the age ranged from 1 year and 1 month to 3 years and 11 months. The dogs were given finrozole at a dose of 3 mg/dog/day from the first day of oestrus signs for 21 days. The development of oestrus signs were followed daily and serum samples collected twice a week. The sexual behaviour of a fertile male and the bitches was recorded. The male was allowed to mount and mate the bitches.

### Results

Oestrus signs disappeared in two bitches of six. Oestrus continued in the rest four. Three of them were mated; two conceived.

According to vaginal cytology, no difference could be detected in oestrus signs in any of the dogs.

Hormonal analyses were carried out at Swedish University of Agricultural Sciences in the Department of Clinical Chemistry Estradiol-17-β (the modified estradiol assay/DPC) and progesterone (Coat-A-Count Progesterone /DPC) were analysed.

The results showed that serum estrogen levels decreased in all bitches during 4-13 days after starting the treatment. Progesterone levels increased in five bitches of six.

No side effects were seen during the treatment.

### Conclusions

The medication lowered the level of serum estradiol in bitches, as expected. In two dogs of six the oestrogen secretion decreased to the extent that the signs of oestrus disappeared. In four dogs the estrogen level decreased but oestrus signs persisted. The weak response was obviously due to the very low dose used in the experiments.

The dose used was determined according to the one used in man. Apparently the reason for the poor response in these experiments was too low dose level. The secretion of estrogen in a bitch during oestrus is much higher than that in man. Thus the dose of aromatase inhibitor needed to lower the secretion in the bitch should be higher.

The described embodiments are illustrative and should not be construed as restrictive.

## Claims

1. A non-therapeutic method of suppressing oestrus in a mammalian non-human animal, comprising administering an effective amount of an aromatase inhibitor of formula (I) 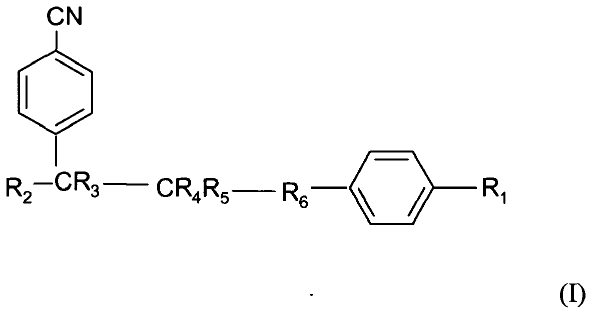 wherein R₁ is hydrogen, methyl, methoxy, nitro, amino, cyano, trifluoromethyl, difluoromethyl, monofluoromethyl or halogen; R₂ is a heterocyclic radical selected from 1-imidazolyl, triazolyl, tetrazolyl, pyrazolyl, pyrimidinyl, oxazolyl, thiazolyl, isoxazolyl and isothiazolyl; R₃ is hydrogen or hydroxy; R₄ is hydrogen; R₅ is hydrogen or hydroxy; or R₄ is hydrogen and R₃ and R₅ combined form a bond; or R₃ is hydrogen and R₄ and R₅ combined form =O; R₆ is methylene, ethylene, -CHOH-, -CH₂CHOH-, -CHOH-CH₂-, -CH=CH- or -C(=O)-; or R₄ is hydrogen and R₅ and R₆ combined is =CH- or =CH-CH₂-; or a stereoisomer, or a non-toxic pharmaceutically acceptable acid addition salt thereof, or a mixture thereof.

2. The method according to claim 1 wherein the aromatase inhibitor is 1-[1-(4-cyanophenyl)-3-(4-fluorophenyl)-2-hydroxypropyl]-1,2,4-triazole, a stereoisomer or a non-toxic pharmaceutically acceptable acid addition salt thereof, or a mixture thereof.

3. The method according to claim 2 wherein the aromatase inhibiting compound is 1-[1-(4-cyanophenyl)-3-(4-fluorophenyl)-2-hydroxypropyl]-1,2,4-triazole, diasteroisomer a + d; the separated isomer a or the separated isomer d.

4. The method according to claim 1, 2 or 3wherein the mammalian animal is a pet.

5. The method according to claim 4, wherein the pet is a canine.

6. The method according to claim 1, 2 or 3 wherein the mammalian animal is a working dog.

## Patentansprüche

1. Ein nicht-therapeutisches Verfahren zum Unterdrücken des Östrus bei einem nichtmenschlichen Säugetier, umfassend das Verabreichen einer effektiven Menge eines Aromatasehemmers der Formel (I), 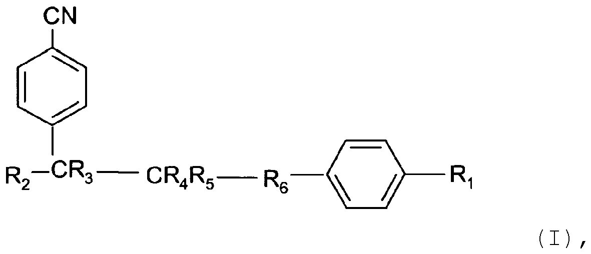 wobei R₁ Wasserstoff, Methyl, Methoxy, Nitro, Amino, Cyano, Trifluormethyl, Difluormethyl, Monofluormethyl oder Halogen ist; R₂ ein heterozyklisches Radikal ist, das aus 1-Imidazolyl, Triazolyl, Tetrazolyl, Pyrazolyl, Pyrimidinyl, Oxazolyl, Thiazolyl, Isoxazolyl und Isothiazolyl ausgewählt wird; R₃ Wasserstoff oder Hydroxy ist; R₄ Wasserstoff ist; R₅ Wasserstoff oder Hydroxy ist; oder R₄ Wasserstoff ist und R₃ und R₅ zusammen eine Bindung bilden; oder R₃ Wasserstoff ist und R₄ und R₅ zusammen =O bilden; R₆ Methylen, Ethylen, -CHOH-, -CH₂CHOH-, -CHOH-CH₂-, -CH=CH- oder -C(=O)- ist; oder R₄ Wasserstoff ist und R₅ und R₆ zusammen =CH- oder =CH-CH₂- sind; oder eines Stereoisomers oder eines nicht-toxischen pharmazeutisch annehmbares Säureadditionssalzes davon oder einer Mischung davon.

2. Das Verfahren gemäß Anspruch 1, wobei der Aromatasehemmer 1-[1-(4-Cyanophenyl)-3-(4-fluorphenyl)-2-hydroxypropyl]-1,2,4-triazol, ein Stereoisomer oder ein nicht-toxisches pharmazeutisch annehmbares Säureadditionssalz davon oder eine Mischung davon ist.

3. Das Verfahren gemäß Anspruch 2, wobei die Aromatasehemmende Verbindung 1-[1-(4-Cyanophenyl)-3-(4-fluorphenyl)-2-hydroxypropyl]-1,2,4-triazol, Diastereoisomer a + d, das getrennte Isomer a oder das getrennte Isomer d ist.

4. Das Verfahren gemäß Anspruch 1, 2 oder 3, wobei das Säugetier ein Haustier ist.

5. Das Verfahren gemäß Anspruch 4, wobei das Haustier ein Hund ist.

6. Das Verfahren gemäß Anspruch 1, 2 oder 3, wobei das Säugetier ein Arbeitshund ist.

## Revendications

1. Méthode non thérapeutique de suppression de l'oestrus chez un mammifère non humain, comprenant l'administration d'une quantité efficace d'un inhibiteur d'aromatase de formule (I) 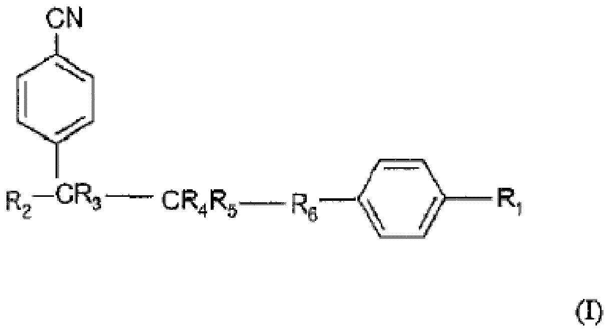 dans laquelle R₁ est un hydrogène, un méthyle, un méthoxy, un nitro, un amino, un cyano, un trifluorométhyle, un difluorométhyle, un monofluorométhyle ou un halogène ; R₂ est un radical hétérocyclique choisi parmi le 1-imidazolyle, le triazolyle, le tétrazolyle, le pyrazolyle, le pyrimidinyle, l'oxazolyle, le thiazolyle, l'isoxazolyle et l'isothiazolyle ; R₃ est un hydrogène ou un hydroxy ; R₄ est un hydrogène ; R₅ est un hydrogène ou un hydroxy ; ou R₄ est un hydrogène et R₃ et R₅ combinés forment une liaison ; ou R₃ est un hydrogène et R₄ et R₅ combinés forment =O ; R₆ est un méthylène, un éthylène, -CHOH-, -CH₂CHOH-, -CHOH-CH₂-, -CH=CH- ou -C(=O)- ; ou R₄ est un hydrogène et R₅ et R₆ combinés sont =CH- ou =CH-CH₂- ; ou l'un de ses stéréoisomères, ou l'un de ses sels d'addition à un acide non toxiques pharmaceutiquement acceptables ou leurs mélanges.

2. Méthode selon la revendication 1, dans laquelle l'inhibiteur d'aromatase est le 1-[1-(4-cyanophényl)-3-(4-fluorophényl)-2-hydroxypropyl]-1,2,4-triazole, l'un de ses stéréoisomères, ou l'un de ses sels d'addition à un acide non toxiques pharmaceutiquement acceptables ou leurs mélanges.

3. Méthode selon la revendication 2, dans laquelle le composé inhibiteur d'aromatase est le 1-[1-(4-cyanophényl)-3-(4-fluorophényl)-2-hydroxypropyl]-1,2,4-triazole, diastéréoisomères a + d ; l'isomère a séparé ou l'isomère d séparé.

4. Méthode selon la revendication 1, 2 ou 3, dans laquelle le mammifère est un animal de compagnie.

5. Méthode selon la revendication 4, dans laquelle l'animal de compagnie est un chien.

6. Méthode selon la revendication 1, 2 ou 3, dans laquelle le mammifère est un chien de travail.
